# EUROPEAN PATENT APPLICATION

(11) **EP 4 461 305 A2**
(43) Date of publication of application: **13.11.2024**
(21) Application number: 24167611.3
(22) Date of filing: 28.03.2024
(51) Int. Cl.: A61K 38/16, A23L 33/18, A61P 3/10, C07K 7/06

(54) **COMPOSITION FOR LOWERING BLOOD SUGAR CONTAINING GREEN TEA PEPTIDE COMPOSITION**

(30) Priority: 19.04.2023 KR 20230051410
(71) Applicant: Amorepacific Corporation, Seoul 04386 (KR)
(72) Inventor: Jeong, Hyun Woo, 17074 Yongin (KR); Chung, Jinoh, 17074 Yongin (KR); Kim, Wanki, 17074 Yongin (KR); Roh, Jong Hwa, 17074 Yongin (KR)
(74) Representative: Dr. Schön, Neymeyr & Partner Patentanwälte mbB

(57) **Abstract**

The present disclosure relates to a green tea peptide composition for use in lowering blood sugar, and specifically, the green tea peptide composition according to the present disclosure exhibits a blood sugar-lowering effect through excellent stimulation of glucose transport in adipocytes, inhibition of gluconeogenesis in hepatocytes, and DPP-4 inhibitory activity, and can be applied to various health functional food compositions and pharmaceutical compositions.

## Description

### Field of the Invention

The present disclosure relates to a composition for lowering blood sugar comprising a green tea peptide composition.

### Description of the Related Art

The tea tree is one of 82 species classified in the genus Camellia and is currently cultivated in over 50 countries, mainly in Asia, Africa, South America, Oceania, etc. Types of tea are largely divided into non-fermented tea, semi-fermented tea, fermented green tea, and post-fermented tea according to the processing method of the tea tree leaves. Among these, non-fermented tea is made by inactivating the polyphenol oxidase contained in the tea tree through heat treatment. Compared to other teas, it contains a lot of polyphenols such as flavonols, flavanones, flavonoids, etc., and shows strong antioxidant power. These substances account for approximately 30% of the dry weight of tea.

As the pharmacological mechanisms of various substances contained in green tea have been gradually revealed, their value has been recognized by the general public. In particular, polyphenols, the main substances of green tea are attracting great attention as it has been proven to have antioxidant, anti-cancer, blood cholesterol-lowering, anti-aging, heavy metal detoxification, cavity prevention, and bad breath removal effects.

Among the substances contained in plants, peptides are stable against oxidation and are expected to have high skin efficacy due to their simple structure. Peptides in plants act as signaling substances and are known to be particularly involved in plant growth, differentiation, and responses to external stimuli.

Meanwhile, since diabetes is almost impossible to cure by treatment after onset, the purpose of diabetes treatment is mainly preventive treatment to improve symptoms caused by diabetes or prevent acute and chronic complications related to diabetes. This includes eliminating symptoms caused by hyperglycemia, preventing and treating acute complications such as diabetic ketoacidosis and hyperglycemic hyperosmolar syndrome caused by hypoglycemia or severe insulin deficiency, retinopathy, nephropathy, neuropathy, atherosclerosis, cerebrovascular disease, and coronary artery disease.

Most diabetic patients in Korea have type 2 diabetes, which is a metabolic disease characterized by hyperglycemia and is caused by decreased insulin secretion from pancreatic beta cells or increased insulin resistance in peripheral tissues due to genetic, metabolic, and environmental factors. Such high blood sugar damages pancreatic beta cells and causes cell death, so effective control of blood sugar is most important for treating type 2 diabetes.

Therefore, lowering blood sugar is most important in treating diabetes. Controlling blood sugar levels in diabetic patients generally involves a combination of drug treatment and dietary therapy, and as problems related to drug use have become more prominent, research reports on the pharmacological efficacy of natural products and functional ingredients have been actively conducted in recent years.

### SUMMARY OF THE INVENTION

The purpose of the present invention is to provide a green tea protein and a composition that exhibits an excellent blood sugar-lowering effect by containing a green tea peptide as an active ingredient having a novel amino acid sequence isolated and purified through culturing of specific vegetable lactic acid bacteria.

In order to achieve the above purpose, an embodiment of the present invention is a composition for lowering blood sugar, which comprises a green tea peptide composition, wherein the green tea peptide composition comprises one or more green tea peptides comprising an amino acid sequence selected from the group consisting of SEQ ID NO: 1 to SEQ ID NO: 7.

The green tea peptide composition according to the present disclosure exhibits a blood sugar-lowering effect through excellent stimulation of glucose transport in adipocytes, inhibition of gluconeogenesis in hepatocytes, and DPP-4 inhibitory activity, and can be applied to various health functional food compositions and pharmaceutical compositions.

### BRIEF DESCRIPTION OF THE DRAWINGS

FIG. 1 schematically shows the manufacturing process of green tea peptide according to an embodiment of the present disclosure.
FIG. 2 shows the results of confirming the effect of activating insulin-dependent glucose transport in adipocytes by concentrations of the green tea peptide composition (GTP) according to Test Example 1 (***P < 0.001 vs. INS, **P < 0.01 vs. INS, ###P < 0.05 vs. (-)).
FIGs. 3A and 3B show the results of confirming the effect of inhibiting gluconeogenesis-related gene expression by concentrations of the green tea peptide composition (GTP) according to Test Example 2 (***P < 0.001 vs. DEX, **P < 0.01 vs. DEX).
FIG. 4 shows the results of confirming the effect of inhibiting gluconeogenesis in hepatocytes by concentrations of the green tea peptide composition (GTP) according to Test Example 2 (***P < 0.001 vs. DEX, **P < 0.01 vs. DEX, *P < 0.05 vs. DEX).
FIG. 5 shows the results of confirming the effect of inhibiting DDP-4 expression in hepatocytes by concentrations of the green tea peptide composition (GTP) according to Test Example 3 (***P < 0.001 vs. LPS, **P < 0.01 vs. LPS, *P < 0.05 vs. LPS).
FIG. 6 shows the results of confirming the effect of the green tea peptide composition (GTP) according to Test Example 3 on inhibiting DDP-4 expression in macrophages (***P < 0.001 vs. LPS, **P < 0.01 vs. LPS, *P < 0.05 vs. LPS).
FIG. 7 shows the results of comparing and confirming the effect of green tea peptides on promoting glucose transport in adipocytes according to the processing method according to Test Example 4 (**P < 0.01 vs. INS, *P < 0.05 vs. INS).
FIGs. 8A and 8B show the results of comparing and confirming the effects of the green tea peptides on inhibiting gluconeogenesis gene expression in hepatocytes according to the processing method according to Test Example 4 (***P < 0.001 vs. DEX, **P < 0.01 vs. DEX, *P < 0.05 vs. DEX).
FIG. 9 shows the results of comparing and confirming the effects of the green tea peptides on inhibiting DDP-4 expression in hepatocytes according to the processing method according to Test Example 4 (***P < 0.001 vs. DEX, **P < 0.01 vs. DEX).
FIG. 10 shows the results of comparing the similarity between *L. paracasei* and *Lactiplantibacillus plantarum.*
FIGs. 11A and 11B show the results of analyzing the molecular weight of green tea protein digests by *L. paracasei* and *Lactiplantibacillusplantarum,* respectively.
FIG. 12 shows the results of comparing the effects of the green tea peptide composition (GTP) according to Test Example 5 on promoting glucose transport in adipocytes by molecular weight fraction (***P < 0.001 vs. INS, **P < 0.01 vs. INS, *P < 0.05 vs. INS).

### DETAILED DESCRIPTION OF THE INVENTION

Hereinafter, the present invention will be described in detail.

In this specification, "green tea (*Camellia sinensis*)" is an evergreen broad-leaved shrub belonging to the Camellia family, and tea from which the leaves are dried is used in various fields. In particular, it is known to exhibit antioxidant, anti-cancer, reduction of blood lipids and promotion of blood circulation effects in the cardiovascular system. The green tea may comprise at least one selected from the group consisting of leaves, flowers, stems, fruits, roots, stems, and heartwoods of roots of green tea, and may preferably be leaves.

In this specification, "active ingredient" refers to an ingredient that can exhibit the desired activity alone or can exhibit activity in combination with a carrier that is not active on its own.

In one aspect, the present invention may relate to a composition for lowering blood sugar, comprising a green tea peptide composition as an active ingredient.

In another aspect, the present invention may relate to a method for lowering blood sugar, which comprises administering an effective amount of a green tea peptide composition to a subject in need thereof.

In another aspect, the present invention may relate to a use of a green tea peptide composition for preparing a composition for lowering blood sugar.

In another aspect, the present invention may relate to a green tea peptide composition for use in lowering blood sugar.

In another aspect, the present invention may relate to a non-therapeutic use of a green tea peptide composition for lowering blood sugar.

In one embodiment, the green tea peptide composition may comprise one or more green tea peptides comprising an amino acid sequence selected from the group consisting of SEQ ID NO: 1 to SEQ ID NO: 7. Specifically, the green tea peptides may comprise an amino acid sequence selected from the group consisting of AYKRRKGKFA (SEQ ID NO: 1), FFFFFFFFFFFFFFFYL (SEQ ID NO: 2), ISKIWNSEVPETEVKNEAESP (SEQ ID NO: 3), PFFCEKMMETN (SEQ ID NO: 4), RFLHERMAYYH (SEQ ID NO: 5), RNLNRLQRLLSMKQEYSPRNHLGSRWREY (SEQ ID NO: 6) and TTSSRKKEKPRRFWNNHEEVFLITTK (SEQ ID NO: 7).

In one embodiment, 60% (w/w) or more, 65% (w/w) or more, 70% (w/w) or more, 75% (w/w) or more, 80% (w/w) or more, 85% (w/w) or more, 90% (w/w) or more, or 95% (w/w) or more of the green tea peptide composition may be composed of one or more types of green tea peptides.

In one embodiment, the green tea peptide composition may be obtained by fermenting green tea protein with vegetable lactic acid bacteria.

In one embodiment, the fermentation may be carried out under conditions of pH 5 to 8. Specifically, the fermentation may be carried out at pH 5 or higher, pH 5.2 or higher, pH 5.4 or higher, pH 5.6 or higher, pH 5.8 or higher, pH 6 or higher, pH 6.2 or higher, pH 6.4 or higher, pH 6.6 or higher, pH 6.8 or higher, pH 7 or higher, pH 7.2 or higher, pH 7.4 or higher, pH 7.6 or higher, or pH 7.8 or higher, and the fermentation also may be carried out at pH 8 or lower, pH 7.8 or lower, pH 7.6 or lower, pH 7.4 or lower, pH 7.2 or lower, pH 7 or lower, pH 6.8 or lower, pH 6.6 or lower, pH 6.4 or lower, pH 6.2 or lower, pH 6 or lower, pH 5.8 or lower, pH 5.6 or lower, pH 5.4 or lower, or pH 5.2 or less. Preferably, the fermentation may be carried out at pH 6.8.

In one embodiment, the fermentation may be carried out at 25 °C to 45 °C. Specifically, the fermentation may be carried out at 25 °C or higher, 27 °C or higher, 29 °C or higher, 31 °C or higher, 33 °C or higher, 35 °C or higher, 37 °C or higher, 39 °C or higher, 41 °C or higher, or 43 °C or higher, and the fermentation also may be carried out at 45°C or lower, 43°C or lower, 41°C or lower, 39°C or lower, 37°C or lower, 35°C or lower, 33°C or lower, 31°C or lower, 29°C or lower, or 27°C or lower. Preferably, the fermentation may be carried out at 37°C.

In one embodiment, the fermentation may be carried out for 24 to 72 hours. Specifically, the fermentation may be carried out for 24 hours or more, 26 hours or more, 28 hours or more, 30 hours or more, 32 hours or more, 34 hours or more, 36 hours or more, 38 hours or more, 40 hours or more, 42 hours or more, 44 hours or more, 46 hours or more, 48 hours or more, 50 hours or more, 52 hours or more, 54 hours or more, 56 hours or more, 58 hours or more, 60 hours or more, 62 hours or more, 64 hours or more, 66 hours or more, 68 hours or more, or 70 hours or more, and the fermentation also may be carried out for 72 hours or less, 70 hours or less, 68 hours or less, 66 hours or less, 64 hours or less, 62 hours or less, 60 hours or less, 58 hours or less, 56 hours or less, 54 hours or less, 52 hours or less, 50 hours or less, 48 hours or less, 46 hours or less, 44 hours or less, 42 hours or less, 40 hours or less, 38 hours or less, 36 hours or less, 34 hours or less, 32 hours or less, 30 hours or less, 28 hours or less, or 26 hours or more. Preferably, the fermentation may be carried out for 48 hours.

In one embodiment, the vegetable lactic acid bacteria may be *Lactiplantibacillus plantarum*(formerly named *Lactobacillus plantarum*)*.* More specifically, the vegetable lactic acid bacteria may be *Lactiplantibacillus plantarum* APsulloc 331261 (Korean Culture Center of Microorganisms, accession number KCCM11179P, accession date 20110328).

In one embodiment, the green tea protein may be obtained from the residue of a primary extract obtained by extracting green tea with anhydrous or hydrous C1-C6 lower alcohol.

In one embodiment, the concentration of alcohol in the hydrous C1-C6 lower alcohol may be 20 to 80% (v/v). Specifically, the concentration of alcohol in the hydrous C1-C6 lower alcohol may be 20% (v/v) or more, 22% (v/v) or more, 24% (v/v) or more, 26% (v/v) or more. , 28% (v/v) or more, 30% (v/v) or more, 32% (v/v) or more, 34% (v/v) or more, 36% (v/v) or more, 38% (v) /v) or more, 40% (v/v) or more, 42% (v/v) or more, 44% (v/v) or more, 46% (v/v) or more, 48% (v/v) or more, 50% (v/v) or more, 52% (v/v) or more, 54% (v/v) or more, 56% (v/v) or more, 58% (v/v) or more, 60% (v/ v) or more, 62% (v/v) or more, 64% (v/v) or more, 66% (v/v) or more, 68% (v/v) or more, 70% (v/v) or more, 72% (v/v) or more, 74% (v/v) or more, 76% (v/v) or more, or 78% (v/v) or more, and may also be 80% (v/v) or less, 78%. (v/v) or less, 76% (v/v) or less, 74% (v/v) or less, 72% (v/v) or less, 70% (v/v) or less, 68% (v/v) or less, 66% (v/v) or less, 64% (v/v) or less, 62% (v/v) or less, 60% (v/v) or less, 58% (v/v) or less, 56% ( v/v) or less, 54% (v/v) or less, 52% (v/v) or less, 50% (v/v) or less, 48% (v/v) or less, 46% (v/v) or less , 44% (v/v) or less, 42% (v/v) or less, 40% (v/v) or less, 38% (v/v) or less, 36% (v/v) or less, 34% (v) /v) or less, 32% (v/v) or less, 30% (v/v) or less, 28% (v/v) or less, 26% (v/v) or less, 24% (v/v) or less, or 22% (v/v) or less.

In one embodiment, the hydrous C1-C6 lower alcohol may be a 20 to 80% (v/v) ethanol aqueous solution. Specifically, the hydrous C1-C6 lower alcohol is 20% (v/v) ethanol aqueous solution, 25% (v/v) ethanol aqueous solution, 30% (v/v) ethanol aqueous solution, 35% (v/v) ethanol aqueous solution. , 40% (v/v) ethanol aqueous solution, 41% (v/v) ethanol aqueous solution, 42% (v/v) ethanol aqueous solution, 43% (v/v) ethanol aqueous solution, 44% (v/v) ethanol aqueous solution, 45% (v/v) ethanol aqueous solution, 46% (v/v) ethanol aqueous solution, 47% (v/v) ethanol aqueous solution, 48% (v/v) ethanol aqueous solution, 49% (v/v) ethanol aqueous solution, 50% (v/v) ethanol aqueous solution, 51% (v/v) ethanol aqueous solution, 52% (v/v) ethanol aqueous solution, 53% (v/v) ethanol aqueous solution, 54% (v/v) ethanol aqueous solution, 55% (v/v) ethanol aqueous solution, 56% (v/v) ethanol aqueous solution, 57% (v/v) ethanol aqueous solution, 58% (v/v) ethanol aqueous solution, 59% (v/v) ethanol aqueous solution, 60% (v/v) ethanol aqueous solution, 65% (v/v) ethanol aqueous solution, 70% (v/v) ethanol aqueous solution, 75% (v/v) ethanol aqueous solution, or 80% (v/v) ethanol aqueous solution.

In one embodiment, the green tea protein may be obtained from the residue of a secondary extract which is hydrothermally extracted from the residue of the primary extract.

In one embodiment, the green tea protein may be obtained from the residue of the secondary extract through alkali extraction, filtration, and acid precipitation.

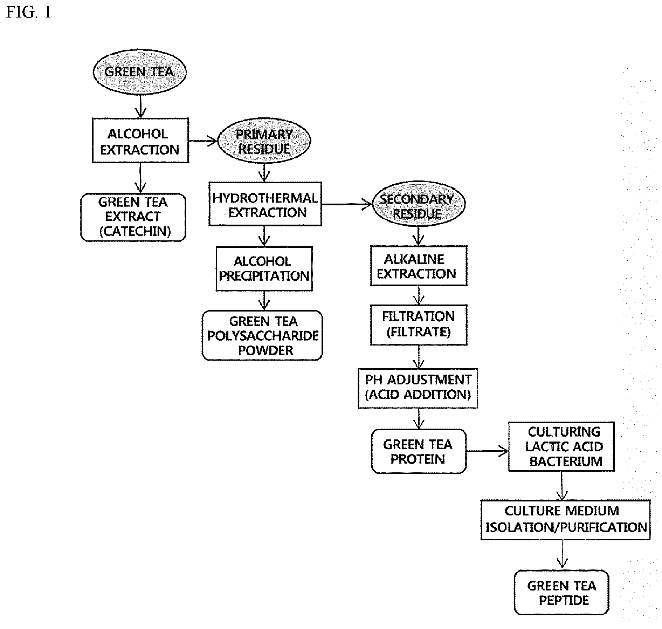

As shown in FIG. 1, the green tea peptide composition according to an embodiment of the present invention may be obtained by first extracting green tea with alcohol, then secondarily extracting the residue remaining with hot water, and then subjecting the residue remaining after the second extracting to alkaline extraction, filtration, and acid precipitation, and then culturing the green tea protein obtained with lactic acid bacteria.

In one embodiment, the green tea peptide composition may promote insulin-dependent glucose transport in adipocytes.

In one embodiment, the green tea peptide composition may inhibit glucose synthesis in hepatocytes.

In one embodiment, the green tea peptide composition may decrease the expression of any one or more of PEPCK and C6Pase.

### Specifically, the green tea peptide composition may inhibit glucose synthesis in hepatocytes by decreasing the expression of any one or more of PEPCK and C6Pase

In one embodiment, the green tea peptide composition may inhibit the degradation of GLP1 (Glucagon-like peptide 1).

In one embodiment, the green tea peptide composition may inhibit the expression of DDP-4 (dipeptidyl peptidase 4) in any one or more cells of hepatocytes and macrophages.

Specifically, the green tea peptide composition may inhibit the degradation of GLP1 by inhibiting the expression of DDP-4 (dipeptidyl peptidase 4) in any one or more cells of hepatocytes and macrophages.

In one embodiment, the green tea peptide composition may be comprised in an amount of 1 to 50 wt% based on the total weight of the composition for lowering blood sugar. Specifically, the green tea peptide composition may be comprised in an amount of 1wt% or more, 3wt% or more, 5wt% or more, 7wt% or more, 10wt% or more, 12wt% or more, 14wt% or more, 16wt% or more, 18wt% or more, 20wt% or more, 22wt% or more, 24wt% or more, 26wt% or more, 28wt% or more, 30wt% or more, 32wt% or more, 34wt% or more, 36wt% or more, 38wt% or more, 40wt% or more, 42wt% or more, 44wt% or more, 46wt% or more or 48wt% or more based on the total weight of the composition for lowering blood sugar, in addition, the green tea peptide composition may be comprised in an amount of 50wt% or less, 48wt% or less, 46wt% or less, 44wt% or less, 42wt% or less, 40wt% or less, 38wt% or less, 36wt% or less, 34wt% or less, 32wt% or less, 30wt% or less, 28wt% or less, 26wt% or less, 24wt% or less, 22wt% or less, 20wt% or less, 18wt% or less, 16wt% or less, 14wt% or less, 12wt% or less, 10wt% or less, 8wt% or less, 7wt% or less, 5wt% or less or 3wt% or less based on the total weight of the composition for lowering blood sugar.

In one embodiment, the green tea peptide composition may be administered or ingested in an amount of 1 to 400 mg/kg/day. Specifically, the green tea peptide composition may be administered or ingested in an amount of 1 mg/kg/day or more, 5 mg/kg/day or more, 10 mg/kg/day or more, 20 mg/kg/day or more, 30 mg/kg/day or more, 40 mg/kg/day or more, 50 mg/kg/day or more, 60 mg/kg/day or more, 70 mg/kg/day or more, 80 mg/kg/day or more, 90 mg/kg/day or more, 100 mg/kg/day or more, 150 mg/kg/day or more, 200 mg/kg/day or more, 250 mg/kg/day or more, 300 mg/kg/day or more or 350 mg/kg/day or more, in addition, the green tea peptide composition may be administered or ingested in an amount of 400 mg/kg/day or less, 350 mg/kg/day or less, 300 mg/kg/day or less, 250 mg/kg/day or less, 200 mg/kg/day or less, 150 mg/kg/day or less, 100 mg/kg/day or less, 90 mg/kg/day or less, 80 mg/kg/day or less, 70 mg/kg/day or less, 60 mg/kg/day or less, 50 mg/kg/day or less, 40 mg/kg/day or less, 30 mg/kg/day or less, 20 mg/kg/day or less, 10 mg/kg/day or less or 5 mg/kg/day or less.

In one embodiment, the composition for lowering blood sugar may be a pharmaceutical or food composition. More specifically, the composition may be a pharmaceutical composition for lowering blood sugar, preventing or treating diabetes, or a health functional food composition for lowering blood sugar.

The formulation of the food composition is not particularly limited, but may be formulated into, for example, liquids such as tablets, granules, pills, powders and drinks, caramels, gels, bars, tea bags, etc. In addition to the active ingredients, the food composition of each formulation may be appropriately selected and mixed by a person skilled in the art according to the formulation or purpose of use with ingredients commonly used in the field without difficulty, and may have a synergistic effect when applied simultaneously with other raw materials.

The composition may be administered in various ways, such as simple ingestion, drinking, injection administration, spray administration, or squeeze administration, etc.

The food composition according to one aspect of the present invention may be various food products such as chewing gum, caramel products, candies, ice cream, and confectionery, etc., beverage products such as soft drinks, mineral water, and alcoholic beverages, etc., health functional food compositions including vitamins and minerals, etc.

The food composition according to one aspect of the present invention may comprise food additives in addition to the active ingredients. Food additives can generally be understood as substances that are added to, mixed with, or infiltrated into food when manufacturing, processing, or preserving food. Since they are consumed daily and for a long period of time with food, their safety must be guaranteed. In the Food Additives Code under the laws of each country that govern the manufacture and distribution of food (in Korea, it is the Food Sanitation Act), food additives with guaranteed safety are limited in terms of ingredients or functions. In the Korea Food Additive Code (Ministry of Food and Drug Safety Notification "Food Additive Standards and Specifications"), food additives are classified into chemical synthetics, natural additives, and mixed preparations in terms of substance. These food additives are classified into sweeteners, flavors, preservatives, emulsifiers, acidulants, thickeners, etc. in terms of function.

Sweeteners are used to impart an appropriate sweetness to food, and both natural and synthetic sweeteners may be used in the food composition according to one aspect of the present invention. Preferably, natural sweeteners are used, and natural sweeteners include sugar sweeteners such as corn syrup solids, honey, sucrose, fructose, lactose, maltose, etc.

Flavors are used to improve taste or aroma, and both natural and synthetic flavors may be used. Preferably, natural products are used. When using natural products, they may serve the purpose of enhancing nutrition in addition to flavor. Natural flavors may be obtained from apples, lemons, tangerines, grapes, strawberries, peaches, etc., or may be obtained from green tea leaves, coriander leaves, bamboo leaves, cinnamon, chrysanthemum leaves, jasmine, etc. Things obtained from ginseng (red ginseng), bamboo shoots, aloe vera, ginkgo nuts, etc. may also be used. Natural flavors may be liquid concentrates or solid extracts. In some cases, synthetic flavors may be used, and esters, alcohols, aldehydes, terpenes, etc. may be used as synthetic flavors.

Preservatives may include calcium sorbate, sodium sorbate, potassium sorbate, calcium benzoate, sodium benzoate, potassium benzoate, EDTA (ethylenediaminetetraacetic acid), etc., and emulsifiers may include acacia gum, carboxymethyl cellulose, xanthan gum, pectin. etc., and acidulants may include acidic acid, malic acid, fumaric acid, adipic acid, gluconic acid, tartaric acid, ascorbic acid, acetic acid, phosphoric acid, etc. In addition to improving taste, acidulants may be added to ensure that the food composition has an appropriate acidity for the purpose of suppressing the growth of microorganisms. Thickening agents, suspending agents, precipitation agents, gel-forming agents, bulking agents, etc. may be used.

In addition to the food additives described above, the food composition according to one aspect of the present invention may comprise biologically active substances or minerals known in the art and whose safety is guaranteed as food additives for the purpose of supplementing and reinforcing functionality and nutrition.

Such biologically active substances may include catechins contained in green tea, vitamins such as vitamin B1, vitamin C, vitamin E, vitamin B12, etc., tocopherol, dibenzoyl thiamine, etc., and minerals may include calcium preparations such as calcium citrate, magnesium preparations such magnesium stearate, iron preparations such as iron citrate, chromium chloride, potassium iodine, selenium, germanium, vanadium, zinc, etc.

The food composition according to one aspect of the present invention may comprise the above-described food additives in an appropriate amount to achieve the purpose of addition depending on the product type.

Regarding other food additives that may be included in the food composition according to one aspect of the present invention, reference may be made to each country's food code or food additive code.

The pharmaceutical composition according to one aspect of the present invention may be prepared into an oral dosage form or a parenteral dosage form depending on the route of administration by a conventional method known in the art, including a pharmaceutically acceptable carrier in addition to the active ingredient. Herein, the route of administration may be any suitable route, including topical route, oral route, intravenous route, intramuscular route, and direct absorption through mucosal tissue, and two or more routes may be used in combination. An example of a combination of two or more routes is a case where two or more dosage forms of drugs according to the administration route are combined, for example, where one drug is administered firstly through an intravenous route and the other drug is administered secondarily through a topical route.

The pharmaceutically acceptable carriers are well known in the art depending on the route of administration or dosage form, and specifically, reference may be made to each country's pharmacopeia, including the "Korean Pharmacopoeia".

When the pharmaceutical composition according to one aspect of the present invention is prepared as an oral dosage form, it may be manufactured in the formulation of powder, granules, tablets, pills, sugar-coated tablets, capsules, solutions, gels, syrups, suspensions, wafers, etc. along with a suitable carrier according to methods known in the art. Examples of suitable carriers at this time include sugars such as lactose, glucose, sucrose, dextrose, sorbitol, mannitol, xylitol, etc., starches such as corn starch, potato starch, wheat starch, etc., cellulose such as cellulose, methylcellulose, ethylcellulose, sodium carboxymethylcellulose, hydroxypropylmethylcellulose, polyvinyl pyrrolidone, water, methyl hydroxybenzoate, propyl hydroxybenzoate, magnesium stearate, mineral oil, malt, gelatin, talc, polyol, vegetable oil, ethanol, glycerol, etc. When formulating, appropriate binders, lubricants, disintegrants, colorants, diluents, etc. may be included as needed. Suitable binders may comprise starch, magnesium aluminum silicate, starch paste, gelatin, methylcellulose, sodium carboxymethylcellulose, polyvinylpyrrolidone, glucose, corn sweetener, sodium alginate, polyethylene glycol, wax, etc. Lubricants may comprise sodium oleate, sodium stearate, magnesium stearate, sodium benzoate, sodium acetate, sodium chloride, silica, talcum, stearic acid, its magnesium and calcium salts, polyethylene glycol, etc. Disintegrants may comprise starch, methylcellulose, agar, bentonite, xanthan gum, alginic acid, or sodium salt thereof, etc. Additionally, diluents may comprise lactose, dextrose, sucrose, mannitol, sorbitol, cellulose, glycine, etc.

When the pharmaceutical composition according to one aspect of the present invention is prepared as a parenteral dosage form, it may be formulated in the form of injections, transdermal administration, nasal inhalation, and suppositories along with a suitable carrier according to methods known in the art. When formulated as injections, an aqueous isotonic solution or suspension may be used as a suitable carrier. Specifically, an isotonic solution such as PBS (phosphate buffered saline) containing triethanolamine, sterile water for injection, or 5% dextrose may be used. When formulated for transdermal administration, it may be formulated in the form of ointments, creams, lotions, gels, external solutions, paste preparations, liniment preparations, aerosol preparations, etc. In the case of nasal inhalation, it may be formulated in the form of an aerosol spray using suitable propellants such as dichlorofluoromethane, trichlorofluoromethane, dichloro tetrafluoromethane, carbon dioxide, etc. When formulated as a suppository, Witepsol, Tween 61, polyethylene glycols, cocoa fat, laurinum, polyoxyethylene sorbitan fatty acid esters, polyoxyethylene stearates, sorbitan fatty acid esters, etc. may be used as the carrier.

The application amount or dosage of the pharmaceutical composition according to one aspect of the present invention will vary depending on the age, gender, weight, pathological condition, and severity of the subject to be administered, route of administration, or the judgment of the prescriber. Determination of active ingredient dosage based on these factors is within the level of a person skilled in the art.

In addition to the active ingredient, the composition for lowering blood sugar according to one aspect of the present invention may further comprise any compounds or natural extracts whose safety has already been verified and which are known to have the corresponding activity in the art to increase and reinforce the effect of lowering the blood sugar level or to improve the convenience of taking or ingesting by adding similar activities such as blood pressure regulating activity. These compounds or extracts include compounds or extracts listed in compendial documents such as the Pharmacopoeia of each country (in Korea, the "Korean Pharmacopoeia"), the Code of Health Functional Foods of each country (in Korea, it is the "Standards and Specifications for Health Functional Foods" notified by the Ministry of Food and Drug Safety), compounds or extracts that have received product approval under each country's laws governing the manufacture and sale of pharmaceuticals (in Korea, this is the "Pharmaceutical Affairs Act"), compounds or extracts whose functionality is recognized under each country's laws governing the manufacture and sale of health functional foods (in Korea, this is the "Health Functional Foods Act").

For example, according to Korea's "Health Functional Foods Act," these compounds or extracts may include complexes such as Garcinia cambogia bark extract, conjugated linolenic acid (free fatty acid), conjugated linolenic acid (triglyceride), green tea extract, chitosan, *Lactobacillus gasseri* BNR17, L-carnitine tartrate, green mate extract, green coffee bean extract, perilla leaf extract, soybean germ extract, etc., complexs (xanthigen) such as gestalt leaf alcohol extract powder, lactoferrin (milk purified protein), lemon balm extract mixed powder, mate hot water extract, seaweed, etc., complex extracts such as fermented vinegar pomegranate complex, pu-erh tea extract, Seomoktae (black soybean) peptide complex, vegetable oil diglyceride, wild mango seed extract, medium chain fatty acid (MCFA)-containing oil, coleus forskohlli extract, chitooligosaccharide, finger root extract powder, hibiscus, etc., which are recognized for their `body fat reduction' functionality, GABA-containing powder derived from L-glutamic acid, katsuobushi oligopeptide, natto bacteria culture powder, salmon peptide, olive leaf extract, sardine peptide, casein hydrolyzate, coenzyme Q10, grape seed enzymatically decomposed extract powder, Haitai oligopeptide, etc., which are recognized for their 'blood pressure regulation' functionality, DHA concentrated oil, globin hydrolyzate, indigestible maltodextrin, bamboo leaf. extract, vegetable oil diglyceride, sardine refined fish oil, refined squid oil, etc., which are recognized for their `improvement of neutral fat in blood' functionality, L-arabinose, nopal extract, cinnamon extract powder, guava leaf extract, freeze-dried silkworm powder, hemp alcohol extract, Banaba leaf extract, upper extract, etc., which are recognized for their 'regulation of the blood sugar levels' functionality, fermentation-generated amino acid complex, Hovenia vulgaris fruit extract, Rhodiola rosea extract, etc., which are recognized for their 'alleviation of fatigue' functionality, L-theanine, Asiaganda extract, milk protein hydrolysate, Gynectarsia leaf extract, which are recognized for their 'anti-stress' functionality.

As an example, the present invention may provide the following embodiments.

A first embodiment may provide a green tea peptide composition for use in lowering blood sugar, which comprises one or more green tea peptides comprising an amino acid sequence selected from the group consisting of SEQ ID NO: 1 to SEQ ID NO: 7.

A second embodiment may provide the green tea peptide composition according to the first embodiment, wherein the green tea peptide composition is obtained by fermenting green tea protein with vegetable lactic acid bacteria, preferably, wherein the vegetable lactic acid bacteria is *Lactiplantibacillus plantarum.*

A third embodiment may provide the green tea peptide composition according to one or more of the first and second embodiments, wherein the green tea protein is obtained from the residue of a primary extract obtained by extracting green tea with anhydrous or hydrous C1-C6 lower alcohol, preferably, wherein the concentration of alcohol in the hydrous C1-C6 lower alcohol is 20 to 80% (v/v), more preferably, herein the hydrous C1-C6 lower alcohol is a 20 to 80% (v/v) ethanol aqueous solution.

A fourth embodiment may provide the green tea peptide composition according to one or more of the first to third embodiments, wherein the green tea protein is obtained from a residue of a secondary extract which is hydrothermally extracted from the residue of the primary extract.

A fifth embodiment may provide the green tea peptide composition according to one or more of the first to fourth embodiments, wherein the green tea peptide composition promotes insulin-dependent glucose transport in adipocytes; wherein the green tea peptide composition inhibits glucose synthesis in hepatocytes; wherein the green tea peptide composition decreases the expression of any one or more of PEPCK and C6Pase; wherein the green tea peptide composition inhibits the degradation of GLP1 (Glucagon-like peptide 1); and/or wherein the green tea peptide composition inhibits the expression of DDP-4 (dipeptidyl peptidase 4) in any one or more cells of hepatocytes and macrophages.

A sixth embodiment may provide the green tea peptide composition according to one or more of the first to fifth embodiments, wherein the green tea peptide composition is formulated in the form of a composition and is comprised in an amount of 1 to 50 wt% based on the total weight of the composition, optionally, wherein the green tea peptide composition is formulated in the form of a pharmaceutical composition.

A seventh embodiment may provide the green tea peptide composition according to one or more of the first to sixth embodiments, wherein the green tea peptide composition is administered in an amount of 1 to 400 mg/kg/day.

An eighth embodiment may provide a non-therapeutic health functional food use of a green tea peptide composition for lowering blood sugar, which comprises one or more green tea peptides comprising an amino acid sequence selected from the group consisting of SEQ ID NO: 1 to SEQ ID NO: 7.

A ninth embodiment may provide the non-therapeutic health functional food use of a green tea peptide composition according to eighth embodiments, wherein the green tea peptide composition is obtained by fermenting green tea protein with vegetable lactic acid bacteria, preferably, wherein the vegetable lactic acid bacteria is *Lactiplantibacillus plantarum.*

A tenth embodiment may provide the non-therapeutic health functional food use of a green tea peptide composition according to one or more of the eighth to ninth embodiments, wherein the green tea protein is obtained from the residue of a primary extract obtained by extracting green tea with anhydrous or hydrous C1-C6 lower alcohol, preferably, wherein the concentration of alcohol in the hydrous C1-C6 lower alcohol is 20 to 80% (v/v), more preferably, herein the hydrous C1-C6 lower alcohol is a 20 to 80% (v/v) ethanol aqueous solution.

An eleventh embodiment may provide the non-therapeutic health functional food use of a green tea peptide composition according to one or more of the eighth to tenth embodiments, wherein the green tea protein is obtained from a residue of a secondary extract which is hydrothermally extracted from the residue of the primary extract.

A twelfth embodiment may provide the non-therapeutic health functional food use of a green tea peptide composition according to one or more of the eighth to eleventh embodiments, wherein the green tea peptide composition promotes insulin-dependent glucose transport in adipocytes; wherein the green tea peptide composition inhibits glucose synthesis in hepatocytes; wherein the green tea peptide composition decreases the expression of any one or more of PEPCK and C6Pase; wherein the green tea peptide composition inhibits the degradation of GLP1 (Glucagon-like peptide 1); and/or wherein the green tea peptide composition inhibits the expression of DDP-4 (dipeptidyl peptidase 4) in any one or more cells of hepatocytes and macrophages.

A thirteenth embodiment may provide the non-therapeutic health functional food use of a green tea peptide composition according to one or more of the eighth to twelfth embodiments, wherein the green tea peptide composition is formulated in the form of a composition and is comprised in an amount of 1 to 50 wt% based on the total weight of the composition, optionally, wherein the green tea peptide composition is formulated in the form of a health functional food composition.

A fourteenth embodiment may provide the non-therapeutic health functional food use of a green tea peptide composition according to one or more of the eighth to thirteenth embodiments, wherein the green tea peptide composition is ingested in an amount of 1 to 400 mg/kg/day.

Hereinafter, the contents of the present invention are described in more detail through examples and test examples. However, these examples and test examples are only presented to understand the present invention, and the scope of the present invention is not limited to these examples and test examples. Variations, substitutions, and insertions commonly well-known in the art can be performed, and these are also included within the scope of the present invention.

### [Example 1] Preparation of green tea peptide composition (GTP)

50 kg of green tea (*camelia sinensis,* Agricultural Corporation Osulloc Farm., Ltd.) was placed in a 1-ton extraction tank, and 50% (v/v) ethanol was added thereto 15 times. Then, extraction (primary extraction) at 70°C for 2 hours and filtration were performed to remove catechins, and a residue of the primary extract of green tea was obtained. Purified water was added at a ratio of 15 times into the obtained residual solids of the primary extract of green tea. Water-soluble polysaccharides were then removed through extraction (secondary extract) at 90°C for 3 hours and filtration, and the residue of green tea of secondary extract of green tea. 2% (w/w) NaOH (98%, Youngjin Co., Ltd.) aqueous solution was added to the residual solids of the obtained secondary extract of green tea at a ratio of 10 times, followed by extraction (alkaline extraction) at 70°C for 3 hours and filtration to obtain the filtrate. The obtained filtrate was cooled to room temperature and 35% (w/w) hydrochloric acid (Daejeong Chemical) was added to make the pH between 3.5 and 4.5 or less. After removing the supernatant and washing the precipitate 3 to 7 times with purified water, the precipitate was spray-dried using an Ohkawara OC-16 spray dryer (inlet 220°C, outlet 90°C) to obtain a green tea protein with a crude protein content of more than 50% (w/w). *Lactiplantibacillusplantarum* APsulloc 331261 medium (containing purified water, vitamin solution, amino acid solution, and mineral solution) containing 1% (w/w) of green tea protein was placed in an anaerobic fermenter, and cultured at pH 6.8, and 37°C for 48 hours. The culture was centrifuged at 4°C and 10,000g for 20 minutes (Labogene 1580R (Serial No. KLG4226180220023)) to obtain the supernatant. The obtained supernatant was concentrated in a dry oven at 70°C, and the concentrate was centrifuged at 4°C and 100,000g for 1 hour (Hitachi centrifuge CS150NX) to obtain a supernatant. The obtained supernatant was filtered through membrane filtration (pore size 0.22µm) to obtain a filtrate, and the obtained filtrate was freeze-dried to acquire a green tea peptide composition (Green Tea Peptide; GTP) (green tea peptide content 14% (w/w)).

Meanwhile, sequences of green tea peptides contained in the green tea peptide composition were analyzed through the following steps:
1) The *Lactiplantibacillus plantarum* APsulloc 331261 medium containing the green tea protein was centrifuged to separate the supernatant, followed by membrane filtration and Size Exclusion Chromatography to obtain only peptides fractionated in the section of low-molecular-weight peptides;
2) The peptides obtained in 1) were freeze-dried and de-salted, then dissolved in 0.1% (w/w) formic acid and analyzed by LC-MS/MS,
wherein LC-MS/MS analysis was performed on 3 µg of sample (based on protein quantification), and the equipment and analysis conditions used were as follows;

**[Table 1]**

| LC equiment | UltiMate 3000 RSLC nano system |
|---|---|
| MS equipment | Q-Exactive Orbitrap HF-X mass spectrometer (Thermo Fisher Scientific) |
| Column | (trap) Internal diameter: 75 µm X 2 cm, packed with Acclaim PepMap 100 C18, 3 µm(analytical) Internal diameter: 75 µm X 50 cm, packed with PepMap RSLC C18, 2 µm |
| Mobile phase | (solvent A) 0.1% (w/w) formic acid (FA) in water(solvent B) 0.1% (w/w) FA in acetonitrile |
| Mode | Positive ion mode |
| Collision energy | 27% |

3) Peptide sequences were searched for the spectrum file obtained through LC-MS/MS analysis in 2) using green tea (camelia sinensis [UniProt Proteome ID: UP000327468]) and lactic acid bacteria (*L. plantarum* DSM 20174 [NCBI accession: GCA_014131735.1], *L. plantarum* APsulloc 331261),
wherein the analysis conditions used at this time were as follows.

**[Table 2]**

| Search engine | MS-GF+ |
|---|---|
| Modifications | (fixed) None(variable) oxidation (+15.99) of methionine & acetylation (+42.01) of the peptide N-terminal |
| m/z tolerance | Precursor: ± 10 ppmFragment: ± 20 ppm |
| False discovery rate cut-off | 1% |

The green tea peptide sequences according to an embodiment of the present disclosure analyzed through the above steps are as follows.

**[Table 3]**

| | |
|---|---|
| AYKRRKGKFA | SEQ ID NO: 1 |
| FFFFFFFFFFFFFFFYL | SEQ ID NO: 2 |
| ISKIWNSEVPETEVKNEAESP | SEQ ID NO: 3 |
| PFFCEKMMETN | SEQ ID NO: 4 |
| RFLHERMAYYH | SEQ ID NO: 5 |
| RNLNRLQRLLSMKQEYSPRNHLGSRWREY | SEQ ID NO: 6 |
| TTS SRKKEKPRRFWNNHEEVFLITTK | SEQ ID NO: 7 |

### [Test Example 1] Confirmation of the effect of promoting insulin-dependent glucose transport in adipocytes by treatment with different concentrations

Glucose is used as an energy source in various cells, and in this process, glucose transporters appropriate for the characteristics of each cell are involved. In adipocytes and muscle cells, GLUT4, controlled by insulin, is responsible for the intracellular transport of glucose. GLUT4 is normally stored in the endoplasmic reticulum, but when the insulin signaling system is activated, it immediately moves to the cell membrane and transports glucose. Therefore, if insulin secretion does not occur properly (Type 1 diabetes) or the secreted insulin does not perform its role (Type 2 diabetes), glucose in the blood vessels is not transported to target cells, resulting in high blood sugar levels. This phenomenon is diabetes, and excess glucose produces glycated hemoglobin, etc., which mediates various adult diseases. Type 2 diabetes accounts for more than 90% of diabetic patients in Korea, so improving insulin sensitivity and lowering blood sugar levels is the basis for diabetes prevention and treatment.

To determine whether the green tea peptide composition (GTP) according to an embodiment of the present invention promotes insulin-dependent glucose transport, 3T3-L1, a mouse-derived adipocyte cell line, was purchased from ATCC. The cells were cultured in Dulbecco's Modified Eagle Medium (DMEM; Sigma Aldrich) containing 10% bovine calf serum (Gibco) and 1% penicillin/streptomycin(PS; Sigma Aldrich), and for differentiation into adipocytes, the cells were cultured to 100% confluent and then left for an additional 48 hours to ensure that there were no gaps between cells (post-confluent).

Then, DMEM + 10% fetal bovine serum (FBS; Gibco) supplemented with dexamethasone (1 mM; Sigma Aldrich), insulin (10 mg/ml; Sigma Aldrich), and 3-isobutyl-1-methylxanthine(500 mM; Sigma Aldrich) + 1 % PS medium was treated to initiate differentiation into adipocytes. After 48 hours, the cells were cultured in DMEM + 10% FBS + 1% PS + 10 mg/ml insulin medium for another 10 days to induce complete adipocyte differentiation.

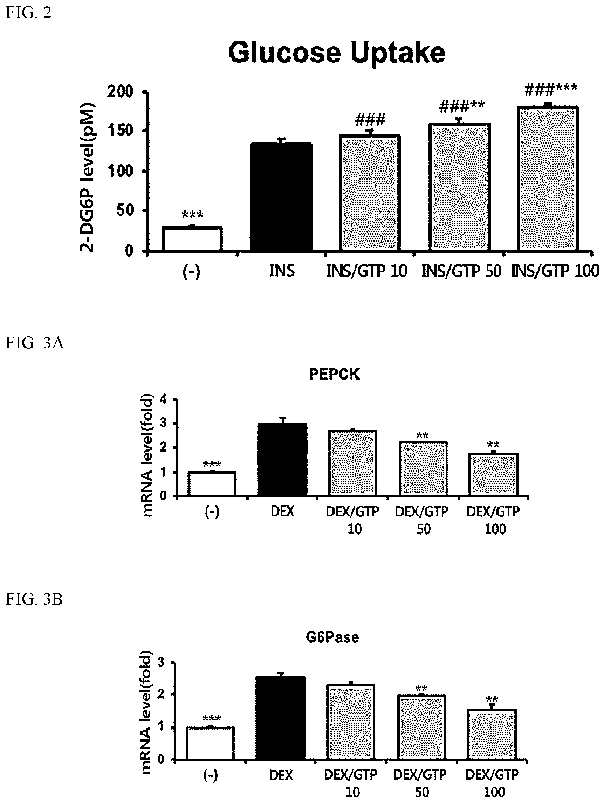

To confirm the effect of promoting glucose transport, the differentiated 3T3-L1 cells were pretreated with the green tea peptide composition of Example 1 at concentrations of 10, 50, and 100 mM for 12 hours, and then treated with 100 nM insulin for 1 hour to activate the insulin signaling system. Intracellular glucose transport was confirmed using 2-deoxyglucose. 2-deoxyglucose can enter the cell through the glucose transporter, but it is not used in glycolysis and accumulates inside the cell. Using these properties, glucose transport capacity can be calculated by measuring the amount of 2-deoxyglucose accumulated inside the cell. The Glucose Uptake Assay Kit(colorimetric; abcam) was used in the experiment, and the results are shown in FIG. 2.

As shown in FIG. 2, it was confirmed that the insulin-dependent glucose transport of adipocytes treated with the green tea peptide composition (GTP) according to Example 1 increased in a concentration-dependent manner.

From this, it can be seen that the green tea peptide composition according to one aspect of the present disclosure will exhibit an excellent blood sugar-lowering effect by promoting insulin-dependent glucose transport in adipocytes.

### [Test Example 2] Confirmation of the effect of inhibiting gluconeogenesis in hepatocytes by treatment with different concentrations of green tea peptide composition (GTP)

When the human body recognizes low blood sugar, it immediately breaks down glycogen in the liver or synthesizes glucose from pyruvic acid. Conversely, when glucose is absorbed through a meal, etc., and the absorbed glucose induces insulin secretion, insulin directs glycogen synthesis and fatty acid synthesis, while gluconeogenesis is suppressed and the absorbed glucose is converted into other nutrients and stored. Blood sugar is controlled through this process, but if insulin resistance occurs, the glucose conversion by insulin does not occur smoothly, resulting in failure to control blood sugar.

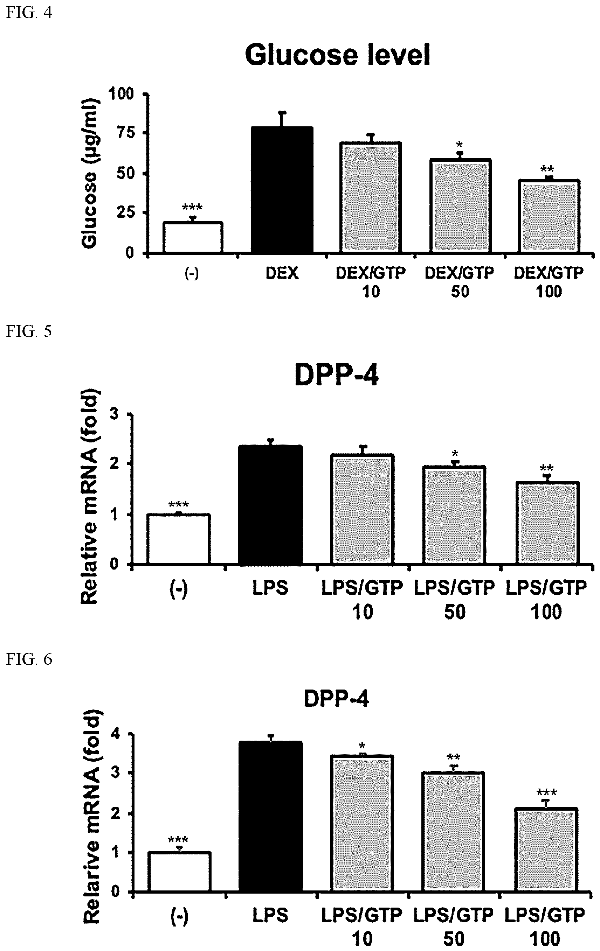

Accordingly, the inhibitory effect of gluconeogenesis in hepatocytes of the green tea peptide composition (GTP) according to an embodiment of the present disclosure was observed. Specifically, the human-derived liver cancer cell line (HepG2) purchased from ATCC was cultured using Roswell Park Memorial Institure-1640 (RPMI) medium (Gibco) containing 10% FBS and 1% PS. For the experiment, the cells were grown in RPMI medium (serum-free) containing only 1% PS, not glucose (Thermo Fisher Scientific) to simulate hypoglycemia and low nutrition. After, the green tea peptide composition of Example 1 was pretreated at concentrations of 10, 50, and 100 mM for 12 hours, and then treated with dexamethasone (10 mM), which induces gluconeogenesis, for an additional 12 hours. After completing the experiment, RNA was extracted from adipocytes using the TaKaRa MiniBEST Universal RNA Extraction Kit (Takara Bio), and then cDNA was synthesized using the RevertAid 1st-strand cDNA Synthesis Kit (Thermo Fisher Scientific). Expression of genes related to gluconeogenesis in hepatocytes was compared using CFX96 thermocycler (Bio-Rad) and related gene primers (Bioneer). After repeating the same experiment, the amount of intracellular glucose was measured using the Glucose Assay Kit (Sigma Aldrich). The results are shown in FIGs. 3A, 3B, and 4.

As shown in FIGs. 3A and 3B, when treated with dexamethasone (DEX), the expression of gluconeogenesis-related genes, PEPCK and G6Pase, significantly increased. On the other hand, when the green tea peptide composition according to an embodiment of the present invention is pretreated, it can be confirmed that the expressions of genes related to gluconeogenesis are reduced by DEX treatment.

Additionally, as shown in FIG. 4, it was confirmed that gluconeogenesis in hepatocytes was effectively inhibited when treated with the green tea peptide composition according to an embodiment of the present invention.

### [Test Example 3] Confirmation of the inhibitory effect of green tea peptide composition (GTP) on DDP-4 expression in hepatocytes and macrophages

When glucose is supplied to the body through food intake, etc., the pancreas secretes Glucagon-like peptide 1 (GLP1) along with insulin. GLP-1 induces a feeling of satiety to stop eating, promotes intestinal peristalsis, mediates digestion, and, like insulin, performs various actions to lower blood sugar. Since GLP-1 is degraded by Dipeptidyl Peptidase 4 (DPP-4), GLP-1 analogs (e.g., liraglutide, semaglutide, exenatide) that DPP-4 does not degrade are widely used as anti-diabetic and anti-obesity treatments. Additionally, there are reports that DPP-4 expression is increased in animals and patients with metabolic diseases such as obesity or diabetes. Accordingly, increasing GLP-1 or inhibiting DPP-4 can be considered necessary to prevent diabetes and various metabolic diseases such as obesity and hyperlipidemia, etc., resulting from it from a long-term perspective. The expression of DPP-4 is promoted by the inflammatory response. Since metabolic disease is a chronic inflammatory state, this promotes the expression and secretion of DPP-4. As a result, the decomposition of GLP-1 is promoted, making blood sugar and metabolic control difficult.

Since DPP-4 is also expressed in the liver, the HepG2 liver cell line was pretreated with the green tea peptide composition according to Example 1 at concentrations of 10, 50, and 100 mM for 12 hours, and then lipopolysaccharide (LPS; 100 ng/ml, Sigma Aldrich) was treated for 24 hours to induce an inflammatory response. Afterward, RNA was extracted and cDNA was synthesized in the same manner as in Test Example 2, and the expression of DPP-4 was confirmed by Q-PCR, and the results are shown in FIG. 5. As previously described, since the expression of DPP-4 increases due to the inflammatory response, the same experiment was performed on RAW 264.7 macrophages (ATCC) in which the inflammatory immune response actively occurs, and the results are shown in FIG. 6.

As shown in FIGs. 5 and 6, regardless of the cell type (HepG2, RAW 264.7), it was confirmed that the green tea peptide composition according to an embodiment of the present invention effectively inhibits the increase in DPP-4 expression caused by LPS.

### [Test Example 4] Comparison and confirmation of the effect of promoting glucose transport in adipocytes of green tea peptide compositions (GTP) according to different manufacturing methods

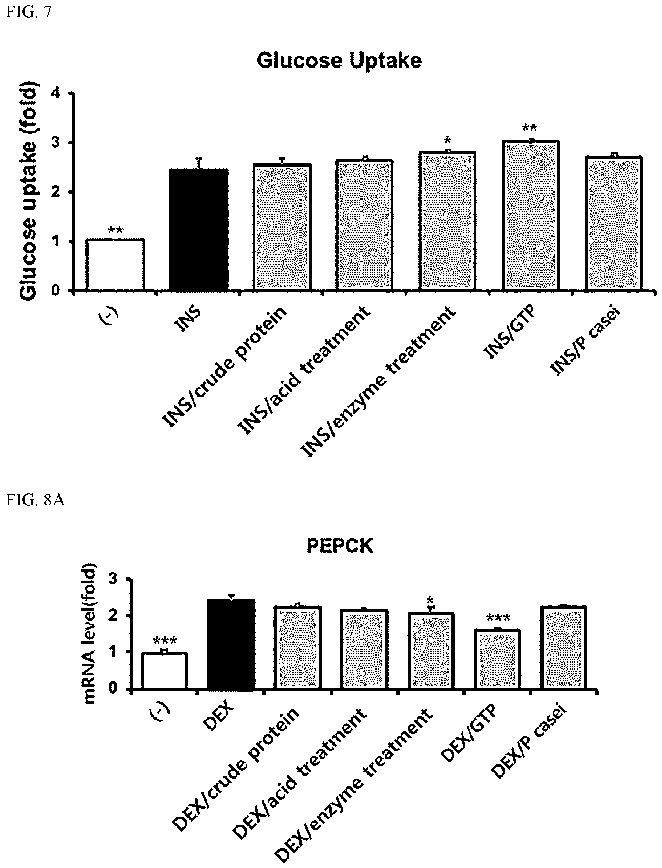

In order to compare the blood sugar-lowering efficacy of green tea peptides according to the manufacturing methods, i) the green tea crude protein obtained during the manufacturing process of the green tea peptide composition of Example 1, ii) the supernatant (green tea protein acid-treated fraction) obtained by preparing the green tea protein obtained during the preparation of the green tea peptide composition of Example 1 at a concentration of 1:40 (w/v) using purified water, adding 35% (w/w) hydrochloric acid to adjust the pH to 5, followed by hydrolysis at 37°C for 6 hours and centrifugation (Labogene 1580R (Serial No. KLG4226180220023)) at 4°C and 10,000g, iii) the supernatant (green tea protein enzyme-treated fraction) obtained by preparing the green tea protein obtained during the preparation of the green tea peptide composition of Example 1 at a concentration of 1:40 (w/v) using sodium phosphate buffer (pH 8), adding bromelain, proteolytic enzyme, at 1% (w/v) compared to green tea protein, followed by hydrolysis at 45°C and pH 6.2 for 24 hours, then heating at 90°C for 30 minutes and centrifugation (Labogene 1580R (Serial No. KLG4226180220023)) at 4°C and 10,000g, and iv) peptide composition prepared in the same manner as in Example 1 except that the animal lactic acid bacterium *Lacticaseibacillus paracasei* (*Lacticaseibacillus paracasei* KCTC 3510 (ATCC strain number: ATCC 25302), Biological Resources Center (KCTC)) was used instead of the *Lactiplantibacillus plantarum* APsulloc 331261 were treated together and the blood sugar-lowering efficacy was compared. The specific experimental methods were conducted in the same manner as in Test Examples 1 to 3 above except treatment with green tea crude protein, green tea protein acid-treated fraction, green tea protein enzyme (bromelain)-treated fraction, *Lactiplantibacillus paracasei* fermented peptide composition, which is an animal lactic acid bacterium, and green tea peptide composition according to Example 1, respectively. The results are shown in FIGs. 7, 8A, 8B, and 9, respectively.

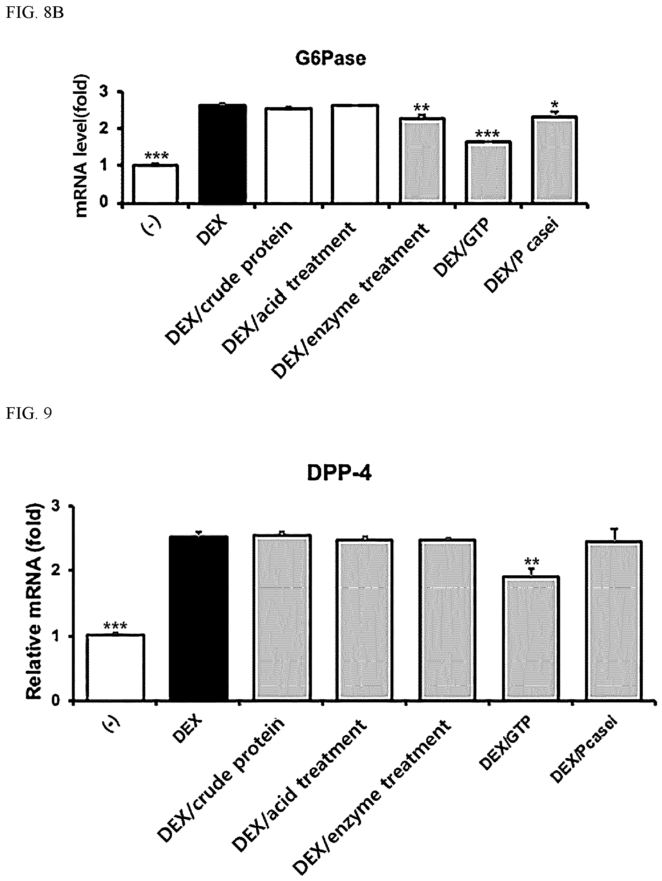

From the results of FIG. 8, it confirmed that the green tea crude protein, acid-degraded peptide, enzyme-degraded peptide, and animal lactic acid bacteria-fermented peptide composition show no effect or very minimal effect. In contrast, the green tea peptide composition according to an embodiment of the present invention exhibited significant and excellent effects of promoting intracellular glucose transport in adipocytes (FIG. 7), suppressing gluconeogenesis gene expression in hepatocytes (FIGs. 8A and 8B), and suppressing DDP-4 expression in hepatocytes (FIG. 9).

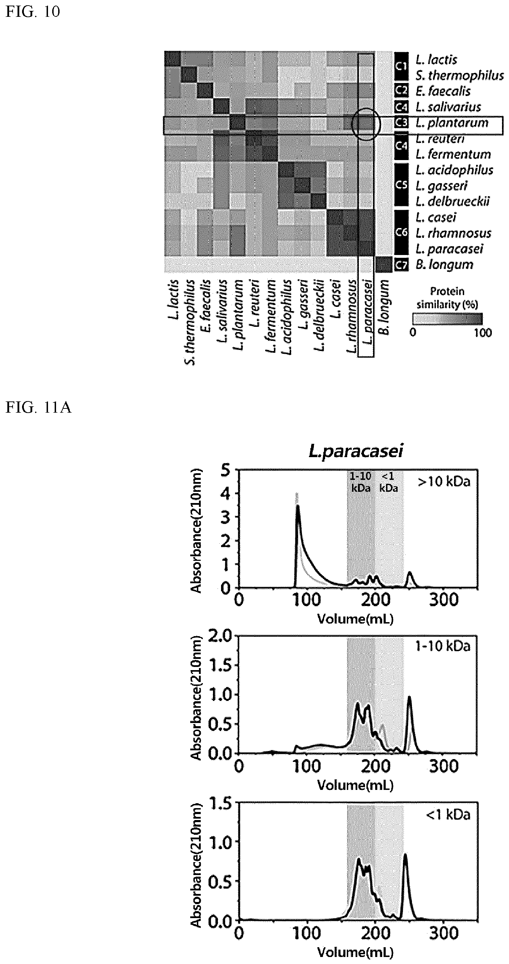

Meanwhile, FIG. 10 shows the results of analyzing the similarity between *Lactiplantibacillus plantarum* and *Lactiplantibacillus paracasei.* Similarity analysis was calculated by downloading the genome information (GenBank) for each strain from NCBI (https://www.ncbi.nlm.nih.gov/genbank/), leaving only genetic information of the annotated conserved protein, discarding the rest, and calculating the ratio to the total number. At this time, the R program was used to calculate POCP (percentage of conserved proteins) (code source: https://github.com/hoelzer/pocp.git), and the analysis conditions were as follows:
- E-value = 1×e⁻⁵
- Sequence identity = 0.4
- Alignment length = 0.5.

Among these, if two genomes showed more than 50% similarity, they were classified into the same cluster, and *Lactiplantibacillus plantarum* and *L. paracasei* had a similarity of 46.84%. It turned out to be very similar. In this way, in the case of the green tea peptide composition (PCasei) obtained by fermentation with *L. paracasei,* an animal lactic acid bacterium very similar to *Lactiplantibacillus plantarum* used in Example 1, it was confirmed that the effect of promoting intracellular glucose transport in fat calles (FIG. 7), the effect of suppressing gluconeogenesis gene expression in hepatocytes (FIGs. 8A and 8A), and the effect of suppressing DDP-4 expression in hepatocytes (FIG. 9), that is, the blood sugar-lowering effect was not shown. From this, it can be seen that the blood sugar-lowering effect of the green tea peptide composition according to an embodiment of the present invention is obtained through fermentation using *Lactiplantibacillusplantarum,* a vegetable lactic acid bacterium.

### [Test Example 5] Confirmation of blood sugar-lowering effect according to molecular weight of green tea peptide (GTP)

As a result of sequence analysis of the peptide isolated and purified from the green tea peptide composition prepared in Example 1, peptides having the amino acid sequences of SEQ ID NOs: 1 to 7 were identified.

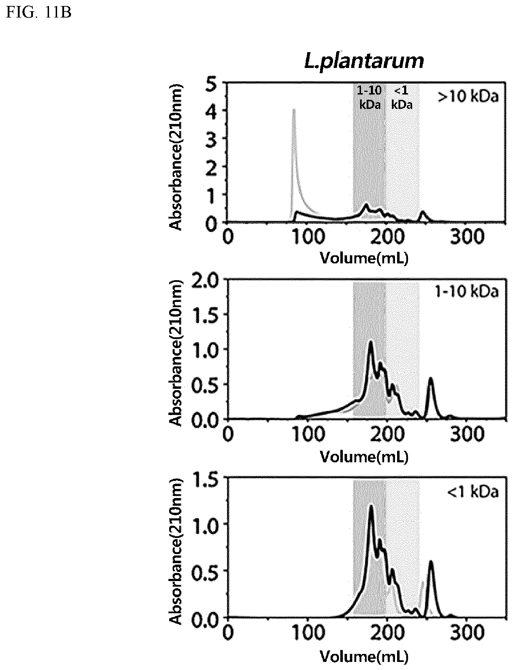

FIGs. 11A and 11B show the results of molecular weight analysis of green tea protein digests by *L. paracasei* and *Lactiplantibacillusplantarum,* respectively. Specifically, molecular weight analysis was performed using size exclusion chromatography using FPLC (Fast Protein Liquid Chromatography), and the specific conditions for FPLC equipment and buffer are as follows:

**[Table 4]**

| Equipment | BioLogic Duoflow Chromatography System | |
|---|---|---|
| Column | Superdex 30 pg (200 ml) | |
| Buffer | 20 mM Tris-HCl, pH 7.0 | |
| Flow rate | 1.5 ml/min | |
| Pressure | 7-9 psi | |
| Sample amount (concentration) | Large molecule fraction (>10 kDa) | 100 mg (50 mg/ml) |
| | Small molecule fraction (≤10 kDa) | 100 mg (50 mg/ml) |
| | Green tea peptide composition (GTP) | 100 mg (50 mg/ml) |

As shown in FIGs. 11A and 11B, the green tea peptide composition obtained through *Lactiplantibacillus plantarum* fermentation is different from the green tea peptide (PCasei) fermented with *L. paracasei* in that it mostly contains small molecular peptides of 10kDa or less.

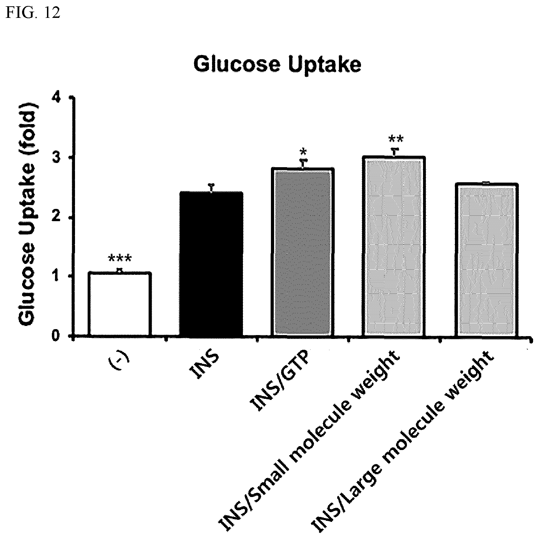

Accordingly, the green tea peptide composition prepared in Example 1 was separated into a small molecule fraction and a large molecule fraction based on a molecular weight of 10 kDa using a dialysis kit (Sigma Aldrich). Each sample was treated at the concentration shown in Table 4, and the efficacy of promoting glucose transport in adipocytes for each sample was compared using the same manner as in Test Example 1. The results are shown in FIG. 12.

As shown in FIG. 12, the small molecule fraction containing a large amount of the novel peptide produced by *Lactiplantibacillus plantarum* fermentation showed an excellent effect of promoting glucose transport in adipocytes. In contrast, the large molecule fraction not containing the novel peptide did not show the effect of promoting glucose transport in adipocytes. From this, it can be seen that the blood sugar-lowering effect of the green tea peptide composition according to an embodiment of the present invention is due to a new peptide degraded by *Lactiplantibacillus plantarum*, a fermented lactic acid bacterium.

### [Accession number]

Name of depository organization: Korea Microbial Conservation Center (overseas)
Accession number: KCCM11179P
Accession date: 20110328

## Claims

1. A green tea peptide composition for use in lowering blood sugar, which comprises one or more green tea peptides comprising an amino acid sequence selected from the group consisting of SEQ ID NO: 1 to SEQ ID NO: 7.

2. The green tea peptide composition according to claim 1, wherein the green tea peptide composition is obtained by fermenting green tea protein with vegetable lactic acid bacteria,
preferably, wherein the vegetable lactic acid bacteria is *Lactiplantibacillusplantarum.*

3. The green tea peptide composition according to claim 1 or 2, wherein the green tea protein is obtained from the residue of a primary extract obtained by extracting green tea with anhydrous or hydrous C1-C6 lower alcohol,
preferably, wherein the concentration of alcohol in the hydrous C1-C6 lower alcohol is 20 to 80% (v/v),
more preferably, herein the hydrous C1-C6 lower alcohol is a 20 to 80% (v/v) ethanol aqueous solution.

4. The green tea peptide composition according to any one of claims 1 to 3, wherein the green tea protein is obtained from a residue of a secondary extract which is hydrothermally extracted from the residue of the primary extract.

5. The green tea peptide composition according to any one of claims 1 to 4,
wherein the green tea peptide composition promotes insulin-dependent glucose transport in adipocytes;
wherein the green tea peptide composition inhibits glucose synthesis in hepatocytes;
wherein the green tea peptide composition decreases the expression of any one or more of PEPCK and C6Pase;
wherein the green tea peptide composition inhibits the degradation of GLP1 (Glucagon-like peptide 1); and/or
wherein the green tea peptide composition inhibits the expression of DDP-4 (dipeptidyl peptidase 4) in any one or more cells of hepatocytes and macrophages.

6. The green tea peptide composition according to any one of claims 1 to 5, wherein the green tea peptide composition is formulated in the form of a composition and is comprised in an amount of 1 to 50 wt% based on the total weight of the composition,
optionally, wherein the green tea peptide composition is formulated in the form of a pharmaceutical composition.

7. The green tea peptide composition according to any one of claims 1 to 6, wherein the green tea peptide composition is administered in an amount of 1 to 400 mg/kg/day.

8. A non-therapeutic health functional food use of a green tea peptide composition for lowering blood sugar, which comprises one or more green tea peptides comprising an amino acid sequence selected from the group consisting of SEQ ID NO: 1 to SEQ ID NO: 7.

9. The non-therapeutic health functional food use of a green tea peptide composition according to claim 8, wherein the green tea peptide composition is obtained by fermenting green tea protein with vegetable lactic acid bacteria,
preferably, wherein the vegetable lactic acid bacteria is *Lactiplantibacillusplantarum.*

10. The non-therapeutic health functional food use of a green tea peptide composition according to claim 8 or 9, wherein the green tea protein is obtained from the residue of a primary extract obtained by extracting green tea with anhydrous or hydrous C1-C6 lower alcohol,
preferably, wherein the concentration of alcohol in the hydrous C1-C6 lower alcohol is 20 to 80% (v/v),
more preferably, herein the hydrous C1-C6 lower alcohol is a 20 to 80% (v/v) ethanol aqueous solution.

11. The non-therapeutic health functional food use of a green tea peptide composition according to any one of claims 8 to 10, wherein the green tea protein is obtained from a residue of a secondary extract which is hydrothermally extracted from the residue of the primary extract.

12. The non-therapeutic health functional food use of a green tea peptide composition according to any one of claims 8 to 11,
wherein the green tea peptide composition promotes insulin-dependent glucose transport in adipocytes;
wherein the green tea peptide composition inhibits glucose synthesis in hepatocytes;
wherein the green tea peptide composition decreases the expression of any one or more of PEPCK and C6Pase;
wherein the green tea peptide composition inhibits the degradation of GLP1 (Glucagon-like peptide 1); and/or
wherein the green tea peptide composition inhibits the expression of DDP-4 (dipeptidyl peptidase 4) in any one or more cells of hepatocytes and macrophages.

13. The non-therapeutic health functional food use of a green tea peptide composition according to any one of claims 8 to 12, wherein the green tea peptide composition is formulated in the form of a composition and is comprised in an amount of 1 to 50 wt% based on the total weight of the composition,
optionally, wherein the green tea peptide composition is formulated in the form of a health functional food composition.

14. The non-therapeutic health functional food use of a green tea peptide composition according to any one of claims 8 to 13, wherein the green tea peptide composition is ingested in an amount of 1 to 400 mg/kg/day.
